# EUROPÄISCHE PATENTANMELDUNG

(11) **EP 0 879 607 A2**
(43) Veröffentlichungstag der Anmeldung: **25.11.1998**
(21) Anmeldenummer: 98106113.8
(22) Anmeldetag: 03.04.1998
(51) Int. Cl.: A61L 29/00, A61L 31/00, A61L 33/00, B05D 5/08, C08J 7/04, C10M 177/00

(54) **Verfahren zur Veminderung des Reibungswiderstandes hydrophiler Oberflächen**

(30) Priorität: 23.05.1997 DE 19721723; 04.03.1998 DE 19809110
(71) Anmelder: HÜLS AKTIENGESELLSCHAFT, 45764 Marl (DE)
(72) Erfinder: Anders, Christine, Dr., 45721 Haltern (DE); Kunz, Roland, 45770 Marl (DE); Zellermann, Marion, 45721 Haltern (DE)

(57) **Zusammenfassung**

Die Erfindung betrifft ein Verfahren zur Verminderung des Reibungswiderstandes von hydrophil beschichteten Polymeroberflächen, bei dem man man die Beschichtung mit einem Komplexbildner für Schwermetallionen behandelt. Die Erfindung betrifft weiterhin Erzeugnisse für technische, biotechnische oder insbesondere medizinische Zwecke oder Teile solcher Erzeugnisse, deren Oberflächen nach dem Verfahren der Erfindung behandelt wurden. Die Erfindung betrifft schließlich die Verwendung solcher für die Herstellung von Apparaten, Geräten und Vorrichtungen für technische, biotechnische oder insbesondere medizinische Zwecke.

## Beschreibung

Die Erfindung betrifft ein Verfahren zur Verminderung der Reibungswiderstandes von hydrophilen Oberflächen durch Behandlung mit einem Komplexbildner. Die Erfindung betrifft weiterhin Erzeugnisse mit nach dem erfindungsgemäßen Verfahren behandelten Oberflächen, die Apparate, Geräte oder Vorrichtungen für technische, biotechnische oder insbesondere medizinische Zwecke sind oder zur Herstellung solcher Apparate, Geräte oder Vorrichtungen dienen.

Die jährlichen Materialverluste durch Verschleiß, d.h. durch Abrasion, Oberflächenzerrüttung und tribochemische Reaktionen, werden allein für Deutschland auf etwa 40 Mrd. DM geschätzt. Neben sorgfältiger, zweckentsprechender Materialauswahl sind Oberflächenbeschichtung und Schmierung übliche Maßnahmen zur Minderung dieser Verluste. Schmierung wird in der Regel durch adsorptive Anbindung von Schichten aus Gleitmitteln oder Schmierölen erreicht. Eine solche adsorptive Anbindung ist jedoch nicht von Dauer.

Neben den aus allen Gebieten der Technik bekannten Verschleißerscheinungen an sich gegeneinander bewegenden Körpern stellen speziell in der Medizintechnik Reibungseffekte zwischen Polymeroberflächen von medizinischen Apparaten, Geräten oder Vorrichtungen, wie Drainageschläuchen, Sonden oder Kathetern, und dem biologischen System, in dem sie angewandt werden, d.h. Organen und Gefäßen, ein großes Problem dar. Die durch Reibung entstehenden Irritationen oder gar Verletzungen von Organen oder Gefäßen sind für-den Patienten zumindest unangenehm und führen darüberhinaus zu immensen Kosten im Gesundheitswesen.

Es besteht ein Zusammenhang zwischen Hydrophilität und Reibungswiderstand einer Oberfläche in dem Sinne, daß reibungsmindernde Beschichtungen auf Geräten oder Vorrichtungen zur Verwendung in wäßrigem Medium sind in der Regel zugleich hydrophil sind. Die Beschichtungen weisen meist einen hohen Wassergehalt auf und verhindern die Adhäsion von reibungserzeugenden und schädlichen festen Partikeln auf der Oberfläche (Lubricating Polymer Surfaces, Herausgeber Y.Ikada und Y.Uyama, Technomic Publishing Co., Inc., S. 45). Dadurch werden bei Kontakt von reibungsmindernd eingestellten Oberflächen von medizinischen Apparaten, Geräten oder Vorrichtungen mit Gewebe und/oder Gefäßwänden Irritationen und Verletzungen verhindert oder wenigstens vermindert. Daneben wird auch die Adsorption von Proteinen aus Gewebeflüssigkeiten durch hydrophile, reibungsmindernde Beschichtungen zurückgedrängt, und ebenso die Thrombusbildung bei Kontakt mit Blut sowie die Kalzifizierung bei Kontakt mit Urin.

Damit diese erwünschten Wirkungen nachhaltig sind, muß eine Ablösung der Beschichtungen verhindert werden. Daher wird eine kovalente Anbindung der reibungsmindernden Beschichtungen angestrebt. Hierzu können hydrophile Polymere unter Zusatz von Initiator thermisch oder photolytisch auf Polymeroberflächen aufgepfropft werden (WO 89/09246).

Eine weitere Methode zur Erzeugung von kovalent gebundenen hydrophilen Schichten auf Polymeroberflächen ist von S.R.Shukla und A.R.Athalye in J.Appl.Polym.Sci., **49** (1993), 2019-24 beschrieben. Dabei werden hydrophile Monomere mit olefinischer Doppelbindung, wie Hydroxyethylmethacrylat, durch photolytisch initiierte Polymerisation auf die Polymeroberfläche gepfropft.

F.Poncin-Epaillard et al. (J.Appl.Polym.Sci. **52** (1994), 1047-61) aktivieren zunächst die Polymeroberflächen durch Einwirkung von Elektronenstrahlen oder durch Plasmabehandlung und beschichten sie dann durch Pfropfpolymerisation von hydrophilen Monomeren mit olefinischer Doppelbindung.

Bei dem Verfahren der deutschen Patentanmeldung 197 15 449.2 werden hydrophile Beschichtungen auf polymeren Substraten dadurch erzeugt,. daß man mindestens ein hydrophiles vinylmonomer strahleninduziert auf einer aktivierten Substratoberfläche pfropfpolymerisiert.

Bei allen genannten Verfahren wird eine Reibungsverminderung im Vergleich zur unbeschichteten Oberfläche beobachtet. Der Effekt ist, je nach Beschichtungsverfahren, mehr oder minder stark ausgeprägt und in quantitativer Hinsicht nicht immer reproduzierbar. Es wäre daher ein Verfahren erwünscht, das den reibungsvermindernden Effekt der Beschichtungen verstärkt und reproduzierbar macht.

### 1. Kurzbeschreibung der Erfindung

Es wurde nun gefunden, daß sich der Reibungswiderstand von hydrophil beschichteten Polymeroberflächen vermindern läßt, wenn man die Beschichtung mit einem Komplexbildner für Schwermetallionen behandelt.

Diese Wirkung des erfindungsgemäßen Verfahrens ist überraschend und nicht zweifelsfrei erklärbar. Möglicherweise enthalten die Beschichtungen Schwermetallionen, die eine "Gleitschicht" aus Wassermolekülen stören, welche sich auf der hydrophilen Beschichtung ausbildet und die primäre Ursache für den reibungsmindernden Effekt ist. Daß der Effekt in quantitativer Hinsicht nicht immer reproduzierbar ist. liegt möglicherweise daran, daß die Schwermetallionen, je nach Einsatzstoffen, Herstell- und gegebenenfalls Nachbehandlungsverfahren mal in größerer, mal in geringerer Menge vorliegen.

### 2. Hydrophil beschichtete Polymeroberflächen

Das erfindungsgemäße Verfahren ist für die Oberflächen der verschiedensten Polymeren geeignet, die nach den verschiedensten Verfahren hydrophil beschichtet sein können. Von den Polymeren seien insbesondere solche genannt, die in der Medizintechnik und in der Biotechnik zum Einsatz kommen, nämlich Polyurethane, Polyvinylchlorid, Polyolefine, Polyamide, Polyester, Polyether, Polyetherblockamide, Polyesterblockamide, Polystyrol, Polycarbonat, Polyorganosiloxan, Polysulfone, Polyisopren, Polyacrylate, Polymethacrylate, Polyisopren. Polytetrafluorethylen und die entsprechenden Copolymeren und Blends sowie natürliche und synthetische Kautschuke.

Das erfindungsgemäße Verfahren ist auf die verschiedensten hydrophilen Beschichtungen anwendbar, z.B. auf alle zuvor erwähnten. Besonders wirkungsvoll ist es bei einer Klasse von Beschichtungen mit ionogenen Gruppen, die zugleich hydrophil und bioaktiv, nämlich in hohem Maße blutverträglich sowie bakterienabweisend sind. Das sind Beschichtungen, die Repetiereinheiten aus Monomeren mit einer oder mehreren olefinischen Doppelbindungen und bestimmten funktionellen Gruppen enthalten, welche einzeln oder in Kombinationen ursächlich für die genannten Wirkungen sind. Diese Beschichtungen sind in der deutschen Patentanmeldung 19720370.1 (O.Z. 5192) im einzelnen beschrieben. Sie enthalten Repetiereinheiten aus einem oder mehreren Monomeren I der 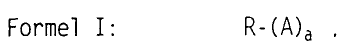 in der
- R: einen ein- oder zweifach olefinisch ungesättigten organischen Rest, beispielsweise einen Kohlenwasserstoffrest, mit der Wertigkeit a bedeutet.
- A: eine Carboxylgruppe -COOH, Schwefelsäuregruppe -OSO₂OH. Sulfonsäuregruppe -SO₃H, Phosphorsäuregruppe -OPO(OH)₂. Phosphonsäuregruppe -PO(OH)₂, Phosphorigsäuregruppe -0P(OH)₂, phenolische Hydroxylgruppe oder ein Salz oder einen Ester einer der genannten Gruppen bezeichnet, und
- a: für eine ganze Zahl von 1 bis 6 steht,
die strahleninduziert auf einer aktivierten Substratoberfläche pfropfpolymerisiert wurden, mit der Maßgabe, daß ein Monomer I, in dem A die Carboxylgruppe -COOH oder ein Salz oder einen Ester der Carboxylgruppe bedeutet, entweder mindestens einen weiteren Rest A mit einer anderen der für A genannten Bedeutungen enthält oder zusammen mit mindestens einem weiteren Monomeren I verwendet wurde, in dem A eine andere der für A genannten Bedeutungen hat.

Unter den Salzen der für A genannten Gruppen werden die Alkalisalze und insbesondere die Natriumsalze bevorzugt.

Das gemeinsame Kennzeichen der Monomeren der Formel I ist, daß sie 1 oder 2 olefinische Doppelbindungen sowie mindestens eine saure hydrophile und bioaktive Gruppe oder ein bestimmtes Derivat, nämlich ein Salz oder einen Ester, einer solchen Gruppe aufweisen.

Die Repetiereinheiten der Beschichtung sind beispielsweise solche aus Monomeren I, die eine Mischung von Monomeren der allgemeinen Formeln II oder III 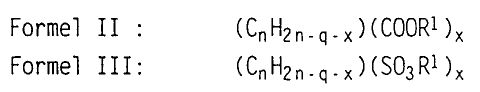 sind. In den Formeln, die in der allgemeinen Formel I eingeschlossen sind, steht
- n: jeweils unabhängig für eine ganze Zahl von 2 bis einschließlich 6;
- x: jeweils unabhängig für 1 oder 2;
- q: jeweils unabhängig für 0 oder 2; und
bedeutet der Rest R¹ jeweils unabhängig -H, ein Äquivalent eines Metallions, insbesondere ein Alkalimetallion, einen Rest eines aliphatischen, cycloaliphatischen oder araliphatischen Alkohols, vorzugsweise eines Alkanols mit 1 bis 6, insbesondere mit 1 bis 4 Kohlenstoffatomen, eines Cycloalkanols mit 5 bis 12 Kohlenstoffatomen, eines Arylalkanols mit 7 bis 10 Kohlenstoffatomen oder eines Alkanols mit Sauerstoff- und/oder Stickstoffatomen in der Kette und bis zu 12 Kohlenstoffatomen, wie -(CH₂-CH₂-O)_{d}-H, -(CH₂-CH(CH₃)-O)_{d}-H, -(CH₂-CH₂-CH₂-O)_{d}-H oder -(CH₂)_{d}-NH₂₋ₑ(R²)ₑ, wobei R² für -CH₃ oder -C₂H₅, d für 1, 2, 3 oder 4 und e für 0, 1 oder 2 steht.

Die Repetiereinheiten der Beschichtung können sich weiterhin von Monomeren I ableiten, die vom Benzol abgeleitete Monomere der allgemeinen Formel 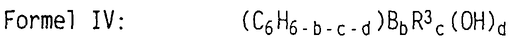 sind, worin
- B: jeweils unabhängig einen ein- oder zweiwertigen geradkettigen oder verzweigten Rest der Formeln (CₙH_{2n-1-q-y})(COOR¹)_{y} oder (CₙH_{2n-1-q-y})(SO₃R¹)_{y} bedeutet, wobei R¹, n und q wie zuvor definert sind und y jeweils unabhängig für 0, 1 oder 2 steht;
- R³: jeweils unabhängig C₁₋₄-Alkyl. -NH₂, -COOH, -SO₃H, -OSO₃H. -OPO(OH)₂, -PO(OH)₂, -OP(OH)₂, -OPO(O⁻)OCH₂-CH₂-N⁺ (CH₃)₃, -PO(O⁻)O-CH₂-CH₂-N⁺(CH₃)₃, -OR(O⁻)OCH₂-CH₂-N⁺(CH₃)₃ oder gegebenenfalls ein Salz, insbesondere ein Alkalisalz, oder einen Ester der genannten Gruppen bedeutet:
- b: für 1, 2, oder 3 steht;
- c: für 0, 1, 2, oder 3 steht; und
- d: für 0, 1, 2, oder 3 steht;
mit der Maßgabe, daß b + c + d ≤ 6, vorteilhaft ≤4 ist.

Die Repetiereinheiten der Beschichtung können auch von Monomeren I abgeleitet sein, die der Formel I entsprechende neutrale oder saure Schwefelsäureester und Salze der letzteren; Sulfonsäuren, deren Salze und Ester; Phosphonsäuren, deren neutrale oder saure Salze, neutrale oder saure Ester sowie Salze der letzteren; Phosphorsäureester, deren neutrale oder saure Salze, neutrale oder saure Ester sowie Salze der letzteren; oder Phosphorigsäuren, deren neutrale oder saure Salze, neutrale oder saure Ester oder Salze der letzteren sind.

Weiterhin können in der Beschichtung Repetiereinheiten von Monomeren I enthalten sein, die der Formel I entsprechende 1 bis 3-wertige (oder -basische) Phenole oder deren Salze sind.

Das erfindungsgemäße Verfahren hat sich besonders bei Beschichtungen bewährt, die einerseits Carboxyl- und/oder Carboxylatgruppen und andererseits Sulfonsäure- und/oder Sulfonatgruppen aufweisen. Es gibt unter dem Aspekt der Verträglichkeit hinsichtlich der genannten Gruppen drei mögliche Zweierkombinationen, nämlich Carboxyl- und Sulfonsäuregruppen, Carboxyl- und Sulfonatgruppen sowie Carboxylat- und Sulfonatgruppen, weiterhin zwei Dreierkombinationen, nämlich Carboxyl-, Carboxylat- und Sulfonatgruppen sowie Carboxyl-, Sulfosäure- und Sulfonatgruppen. Alle diese Kombinationen charakterisieren brauchbare Ausführungsformen des erfindungsgemäßen Verfahrens. Natürlich ist es auch möglich, Monomere einzusetzen, deren funktionelle Gruppen nach der Pfropfpolymerisation verändert werden. So kann man z.B. den Acrylamid-Baustein nachträglich durch Hydrolyse in saurem Medium in den Acrylsäure-Baustein umwandeln. Weiterhin kann man Carboxyl- und Sulfonsäuregruppen durch Neutralisieren (z.B. in Phosphatpuffern) sowie Carbonester- und Sulfosäureestergruppen durch Hydrolyse in Carboxylat- bzw. Sulfonatgruppen überführen.

In den genannten Kombinationen kann das molare Verhältnis von Carboxyl- und/oder Carboxylatgruppen zu Sulfonsäure- und/oder Sulfonatgruppen in der Beschichtung in weiten Grenzen schwanken. Besonders ausgeprägte zellproliferationsinhibierende Eigenschaften werden erzielt, wenn das genannte Verhältnis 0,2 bis 3, vorteilhaft 0,4 bis 3 und insbesondere 0,4 bis 2 beträgt. Die beschichteten Oberflächen zeigen in bemerkenswerter Weise bakterienabweisende. aber zellproliferationsfördernde Eigenschaften, wenn das besagte molare Verhältnis 2 bis 10, vorteilhaft 3 bis 10 und insbesondere 3 bis 5 beträgt. Als zellproliferationsfördernd wird eine Beschichtung dann angesehen, wenn die Haftung und Vermehrung von Säugetierzellen durch die Beschichtung im Vergleich zu der unbeschichteten Oberfläche verbessert oder jedenfalls weniger stark beeinträchtigt wird als die Haftung und Vermehrung von Bakterien.

Von den geeigneten Monomeren der allgemeinen Formeln I bis IV, die die in den Beschichtungen enthaltenen Repetiereinheiten ergeben, seien beispielsweise genannt:

Acrylsäure, Natriumacrylat, Isobutylacrylat, 2-Ethylhexylacrylat, 2-Hydroxyethylacrylat, 2- (2'-Hydroxyethoxy)ethylacrylat, 2-Hydroxy-1-methylethylacrylat, 2-N,N-Dimethylaminoethylacrylat, Methacrylsäure, Natriummethacrylat, n-Propylmethacrylat, 2-Hydroxyethylmethacrylat, 2- (2'-Hydroxyethoxy)ethylmethacrylat, 2-Hydroxy-1-methylethylmethacrylat, 2-N,N-Dimethylaminoethylmethacrylat, Diethylenglykolmethacrylat, Triethylenglykoldiacrylat, 4-Vinylsalicylsäure, Itaconsäure, Vinylessigsäure, Zimtsäure, 4-Vinylbenzoesäure, 2-Vinylbenzoesäure, Sorbinsäure, Coffeinsäure, Maleinsäure, Methylmaleinsäure. Crotonsäure, Isocrotonsäure, Fumarsäure, Dimethylfumarsäure; Natriumallylsulfat, Natriumallylsulfonat, Natriummethallylsulfat, Natriummethallylsulfonat, Natriumvinylsulfonat, Vinylsulfonsäure-2-hydroxyethylester, 4-Vinylbenzolsulfonsäure, Natrium-4-vinylbenzolsulfonat, Natriumvinyltoluolsulfonat; Buten-(2)-diol-(1,4)-diphosphat, Buten-(2)-diol-(1,4)-diphosphonat, die Dinatriumsalze der beiden Phosphate bzw. Phosphonate, Diallylphosphit; 2-Vinylphenol, 2-Allylhydrochinon, 4-Vinylresorcin, m-Hydroxystyrol, o-Hydroxystyrol, p-Hydroxystyrol, Carboxyl-vinylbenzolsulfonsäure.

Schließlich können die Beschichtungen Repetiereinheiten aufweisen, die sich von Monomeren der allgemeinen Formeln V und VI 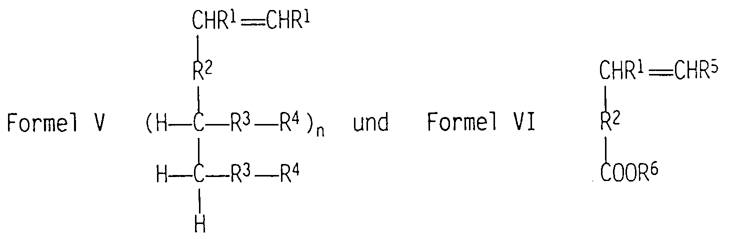 ableiten. In den Formeln und bedeuten
- R¹: Wasserstoff oder den Methylrest,
- R²: einen zweiwertigen organischen Rest, vorzugsweise einen aliphatischen, cycloaliphatischen oder aromatischen Kohlenwasserstoffrest mit bis zu 10 Kohlenstoffatomen, oder eine C-C-Einfachbindung,
- R³: -O- oder -NH-,
- R⁴: Wasserstoff oder den Rest -SO₃⁻Na⁺
- R⁵: Wasserstoff, den Methylrest oder den Rest -R² -COOR⁶,
- R⁶: Wasserstoff oder Na und
- n: 4 oder 5:
mit der Maßgabe, daß mindestens einer der Substituenten R⁴ ein Rest -SO₃⁻Na⁺ ist.

In bevorzugten Monomeren V und VI bedeutet
- R¹: Wasserstoff,
- R²: einen Alkylenrest mit 1 bis 4 Kohlenstoffatomen, einen Phenylenrest oder eine C-C-Einfachbindung,
- R³: -O- oder -NH-,
- R⁴: Wasserstoff oder den Rest -SO₃⁻Na⁺,
- R⁵: Wasserstoff oder den Rest -R²-COOR⁶,
- R⁶: Wasserstoff oder Na und steht
- n: für 4.

Die Herstellung der Monomeren V ist in der deutschen Patentanmeldung (Fall-Nr. 4843) beschrieben. Sie leiten sich von modifizierten Zuckerresten ab. vorzugsweise von Pentosen und insbesondere von Arabinose. Die Zuckerreste enthalten mindestens einen der Reste -O-SO₃⁻Na⁺ (O-Sulfat) oder -NH-SO₃⁻Na⁺ (N-Sulfat), bevorzugt benachbart zu dem Rest R². Sie weisen vorzugsweise 1 bis 4 dieser Reste auf. In einem Zuckerrest können O-Sulfat- und N-Sulfatreste gleichzeitig vorliegen, wobei dann der N-Sulfonatrest bevorzugt benachbart zu zum Rest R² positioniert ist. Alternativ kann der Zuckerrest aber auch ausschließlich eine Art dieser Reste enthalten. z.B. nur O-Sulfatreste.

Die Monomeren VI sind bekannte und gut zugängliche Stoffe, die eine olefinische Doppelbindung und eine oder zwei Carboxyl- oder Carboxylatfunktionen oder Funktionen haben, die nach der Polymerisation in eine Carboxyl- oder Carboxylatfunktion umgewandelt werden können, wie Carboxylgruppen (in Carboxylatgruppen), Carbonester-, Carbonamid- oder Carbonsäureanhydridgruppen. Als Gegenion zur Carboxylatfunktion dienen Natriumionen. Beispiele für geeignete Monomere VI (Meth)acrylsäure, Crotonsäure, 4-Vinylbenzoesäure, Maleinsäure und Fumarsäure sowie deren Natriumsalze.

Das erfindungsgemäße Verfahren ist aber nicht nur auf hydrophile Beschichtungen anwendbar, die durch strahleninduzierte Pfropfung von hydrophilen Monomeren aufgebracht wurden. Die Pfropfung dieser Monomeren kann vielmehr auch durch thermisch initiierten Zerfall von Radikalbildnern, z.B. von organischen Peroxiden oder Makroinitiatoren, ausgelöst werden. Weiterhin können die hydrophilen Beschichtungen auch erzeugt werden, indem hydrophile Polymere strahleninduziert oder thermisch auf Polymersubstrate gepfropft oder aber auf beliebige Substrate in üblicher Weise aufgebracht werden, z.B. durch Spritzen, Rakeln oder Tauchen. Alle diese Beschichtungsverfahren sind dem Fachmann bekannt und nicht Gegenstand der vorliegenden Erfindung.

### 3. Komplexbildner für Schwermetallionen

Die Komplexbildner maskieren die reibungserhöhenden Effekte von Schwermetallionen, die zumeist ungewollt in der Beschichtung vorhanden sind. Von den reibungserhöhenden Schwermetallionen seien u.a. die des Eisens, Mangans, Kobalts und Nickels erwähnt. Sie können z.B. in Spurenmengen mit den Ausgangsstoffen des Beschichtungsverfahrens oder durch Reagentien von Nachbehandlungsverfahren eingeführt worden sein. Die Auswahl der Komplexbildner hängt u.a. von der Beständigkeit der gebildeten Komplexe sowie von der Stärke der Bindung der Schwermetallionen an die bzw. in der jeweiligen Beschichtung ab. Für eine gegebene Beschichtung läßt sich orientierende Versuche unschwer ein erfindungsgemäß wirksamer Komplexbildner ermitteln.

Besonders geeignete Komplexbildner sind Chelatkomplexbildner. Sie bilden mit Schwermetallionen Komplexe, in denen sie zweizähnige Liganden sind. Ihre komplexierende Wirkung ist offenbar stärker als die Wechselwirkung, wie sie beispielsweise zwischen Eisen(II)- oder Eisen(III)ionen und Sulfonat- und/oder Sulfat- und/oder Carboxylatgruppen besteht, die in einer Reihe der oben beschriebenen hydrophilen und auch reibungsvermindernden Beschichtungen vorliegen.

Von den geeigneten Chelatkomplexbildnern seien beispielsweise erwähnt: 1,3-Dicarbonylverbindungen, wie Acetylaceton und Acetessigsäureester; Dimethylglyoxim (oder Diacetyldioxim), Ethylendiamintetraessigsäure (EDTA), Nitrilotriessigsäure (NTA) und Ethylenglykol-bis-(2-aminoethyl)-tetraessigsäure (EGTA). Bevorzugte Chelatkomplexbildner sind NTA und EDTA sowie deren saure oder neutrale Alkali- und Erdalkalimetallsalze.

Man wendet die Chelatkomplexbildner zweckmäßig als wäßrige Lösungen mit einer Konzentration von 0,1 mmol/l bis 0,1 mol/l an. Eine bevorzugte Konzentration ist etwa 10 mmol/l. Die Wirkung tritt rasch ein, es genügen 1 Sekunde bis 10 Minuten, vorzugsweise etwa 10 Sekunden Einwirkungszeit vorzugsweise bei Raumtemperatur, gegebenenfalls bei erhöhter Temperatur bis zu 100°C. Nach der Einwirkung spült man die beschichteten Oberflächen mit kaltem oder warmem Wasser, um den verbliebenen Chelatkomplexbildner zu entfernen.

Den Erfolg des erfindungsgemäßen Verfahrens kann man durch Bestimmung der Reibungskoeffizienten vor und nach der Behandlung messen. Der Reibungskoeffizient kann je nach der Geometrie des beschichteten Gegenstandes nach verschiedenen Verfahren bestimmt werden. Bei flächigen Gebilden ist die Methode der schiefen Ebene anwendbar, so beschrieben in US-A-4 876 126. Diese Methode liefert als Tanges des Winkels, bei dem die Gleitbewegung einsetzt, ein quantitatives Maß für den Haftreibungskoeffizienten. Bei einer anderen, qualitativen Methode wird das Gleitverhalten eines dünneren Schlauches in einem dickeren bestimmt.

### 4. Verwendung der Gegenstände mit vermindertem Reibungswiderstand

Das erfindungsgemäße Verfahren ist für solche Erzeugnisse nützlich. deren Oberfläche sich bei bestimmungsgemäßer Verwendung in wäßrigem Medium in Kontakt mit einer anderen vorzugsweise ebenfalls hydrophilen Oberfläche aus synthetischem oder natürlichem Material befindet und sich gegenüber der anderen Oberfläche bewegt. Die Oberfläche dieser Erzeugnisse kann mit Erfolg nach dem erfindungsgemäßen Verfahren behandelt werden. Solche Erzeugnisse sind u.a. Apparate, Geräte und Vorrichtungen für technische, biotechnische oder insbesondere medizinische Zwecke oder sie sind Teile solcher Apparate. Geräte oder Vorrichtungen. Als Beispiele seien Drainageschläuche. Katheter, Sonden, künstliche Gelenke, Nahtmaterial, Wundabdeckungen oder allgemein Pflaster- und Verbandmaterial genannt.

Das folgende Beispiel soll die Erfindung erläutern, nicht jedoch ihren Anwendungsbereich begrenzen,

### Beispiel

Eine VESTAMID^{(R)}L2101-Folie der Hüls AG (Polylaurinlactam) wurde nach dem Verfahren der deutschen Patentanmeldung 19720370.1 (O.Z. 5192) mit Natriumstyrolsulfonat und Natriumacrylat als hydrophilen Monomeren pfropfbeschichtet. Die Folie war zuvor durch UV-Strahlen einer Wellenlänge von 172 nm aktiviert worden. Die beiden Monomeren wurden im Molverhältnis 1:9 in wäßriger Lösung eingesetzt. Die Pfropfung erfolgte durch Bestrahlung mit UV-Strahlen einer Wellenlänge von 308 nm.

Die beschichtete Folie wurde 10 sec in eine 10mM wäßrige Lösung des Dinatriumsalzes der Ethylendiamintetraessigsäure von Raumtemperatur getaucht. Die Folie wurde dreimal mit Wasser ausgewaschen, um etwaigen überschüssigen Komplexbildner zu entfernen. Danach wurde nach der Methode der schiefen Ebene (analog US-A-4 876 126) die Änderung des Reibungswiderstandes bestimmt. Dazu wurde eine unbeschichtete VESTAMID^{(R)}L2101-Folie auf eine glatte Glasoberfläche aufgeklebt, deren Neigungswinkel gegenüber der Horizontalen stufenlos verstellbar und meßbar war. Die zu prüfende Folie wurde auf die kreisförmige Fläche eines zylinderförmigen Prüfgewichtes (Durchmesser 6 cm, Masse 50 g) geklebt. Beide Folien wurden dann mit einem Pinsel tropfnaß befeuchtet, und das Prüfgewicht wurde mit der beklebten Seite nach unten auf die Gegenfolie aufgesetzt. Der Neigungswinkel der Glasplatte wurde bei 0° beginnend stufenlos und ruckfrei mit etwa 20°/min solange erhöht, bis das aufgesetzte Gewicht in Bewegung geriet. Der zu diesem Zeitpunkt eingestellte Winkel wurde notiert. Sein Tangens gibt den Reibungskoeffizienten der Prüffolie an, der in der folgenden Tabelle vermerkt ist.

| Prüffolie (Gegenfolie jeweils VESTAMID^{(R)}L2101) | Reibungskoeffizient µ |
|---|---|
| unbehandelt | 1,11 |
| beschichtet wie beschrieben | 0,36 |
| beschichtet und behandelt wie beschrieben | 0.08 |

## Patentansprüche

1. Verfahren zur Verminderung des Reibungswiderstandes von hydrophil beschichteten Polymeroberflächen, dadurch gekennzeichnet, daß man die Beschichtung mit einem Komplexbildner für Schwermetallionen behandelt.

2. Verfahren nach Anspruch 1, dadurch gekennzeichnet, daß die Beschichtung eine ionogene Beschichtung mit Repetiereinheiten aus einem oder mehreren Monomeren I der 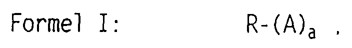 ist, in der
R einen ein- oder zweifach olefinisch ungesättigten organischen Rest, beispielsweise einen Kohlenwasserstoffrest, mit der Wertigkeit a bedeutet,
A eine Carboxylgruppe -COOH, Schwefelsäuregruppe -OSO₂OH. Sulfonsäuregruppe -SO₃H, Phosphorsäuregruppe -OPO(OH)₂, Phosphonsäuregruppe -PO(OH)₂, Phosphorigsäuregruppe -0P(OH)₂, phenolische Hydroxylgruppe oder ein Salz oder einen Ester einer der genannten Gruppen bezeichnet, und
a für eine ganze Zahl von 1 bis 6 steht,
die strahleninduziert auf einer aktivierten Substratoberfläche pfropfpolymerisiert wurden, mit der Maßgabe, daß ein Monomer I, in dem A die Carboxylgruppe -COOH oder ein Salz oder einen Ester der Carboxylgruppe bedeutet, entweder mindestens einen weiteren Rest A mit einer anderen der für A genannten Bedeutungen enthielt oder zusammen mit mindestens einem weiteren Monomeren I verwendet wurde, in dem A eine andere der für A genannten Bedeutungen hat.

3. Verfahren nach Anspruch 2, dadurch gekennzeichnet, daß die Beschichtung Repetiereinheiten aus Monomeren I enthält, die eine Mischung von Monomeren der allgemeinen Formeln II oder III 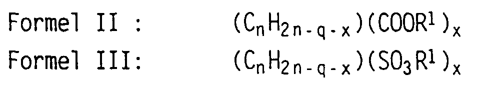 sind, wobei in den Formeln
n jeweils unabhängig für eine ganze Zahl von 2 bis einschließlich 6;
x jeweils unabhängig für 1 oder 2;
q jeweils unabhängig für 0 oder 2; und
bedeutet der Rest R¹ jeweils unabhängig -H, ein Äquivalent eines Metallions, insbesondere ein Alkalimetallion, einen Rest eines aliphatischen, cycloaliphatischen oder araliphatischen Alkohols, vorzugsweise eines Alkanols mit 1 bis 6, insbesondere mit 1 bis 4 Kohlenstoffatomen, eines Cycloalkanols mit 5 bis 12 Kohlenstoffatomen, eines Arylalkanols mit 7 bis 10 Kohlenstoffatomen oder eines Alkanols mit Sauerstoff- und/oder Stickstoffatomen in der Kette und bis zu 12 Kohlenstoffatomen, wie -(CH₂-CH₂-O)_{d}-H, -(CH₂-CH(CH₃)-O)_{d}-H, -(CH₂-CH₂-CH₂-O)_{d}-H oder -(CH₂)_{d}-NH₂₋ₑ(R²)ₑ, wobei R² für -CH₃ oder -C₂H₅, d für 1, 2, 3 oder 4 und e für 0, 1 oder 2 steht.

4. Verfahren nach Anspruch 2, dadurch gekennzeichnet, daß die Beschichtung Repetiereinheiten aus Monomere I enthält, die vom Benzol abgeleitete Monomere der allgemeinen Formel 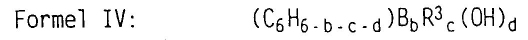 sind, worin
B jeweils unabhängig einen ein- oder zweiwertigen geradkettigen oder verzweigten Rest der Formeln (CₙH_{2n-1-q-y})(COOR¹)_{y} oder (CₙH_{2n-1-q-y})(SO₃R¹)_{y} bedeutet, wobei R¹, n und q wie zuvor definert sind und y jeweils für 0, 1 oder 2 steht;
R³ jeweils unabhängig C₁₋₄-Alkyl, -NH₂, -COOH, -SO₃H, -OSO₃H, -OPO(OH)₂, -PO(OH)₂, -OP(OH)₂, -OPO(O⁻)OCH₂-CH₂-N⁺(CH₃)₃, -PO(O⁻)O-CH₂-CH₂-N⁺(CH₃)₃, -OP(O⁻)OCH₂-CH₂-N⁺(CH₃)₃ oder gegebenenfalls ein Salz, insbesondere ein Alkalisalz, oder einen Ester der genannten Gruppen bedeutet;
b für 1, 2, oder 3 steht;
c für 0, 1, 2, oder 3 steht; und
d für 0, 1, 2, oder 3 steht;
mit der Maßgabe, daß b + c + d ≤ 6, vorteilhaft ≤4 ist.

5. Verfahren nach Anspruch 2, dadurch gekennzeichnet, daß die Beschichtung Repetiereinheiten aus Monomeren I enthält, die der Formel I entsprechende neutrale oder saure Schwefelsäureester und Salze der letzteren; Sulfonsäuren, deren Salze und Ester; Phosphonsäuren, deren neutrale oder saure Salze, neutrale oder saure Ester sowie Salze der letzteren: Phosphorsäureester, deren neutrale oder saure Salze, neutrale oder saure Ester sowie Salze der letzteren: und Phosphorigsäuren, deren neutrale oder saure Salze, neutrale oder saure Ester sowie Salze der letzteren sind.

6. Verfahren nach Anspruch 2, dadurch gekennzeichnet, daß die Beschichtung Repetiereinheiten aus Monomeren I enthält, die 1- bis 3-wertige Phenole oder deren Salze sind.

7. Verfahren nach einem der Ansprüche 2 bis 6, dadurch gekennzeichnet, daß die Beschichtung Repetiereinheiten von Monomeren I enthält, die einerseits Carboxyl- und/oder Carboxylatgruppen und andererseits Sulfonsäure- und/oder Sulfonatgruppen aufweisen.

8. Verfahren nach Anspruch 2, dadurch gekennzeichnet, daß die Beschichtung Repetiereinheiten aus Monomeren I enthält, die eine Mischung aus Monomeren der allgemeinen Formeln V und VI 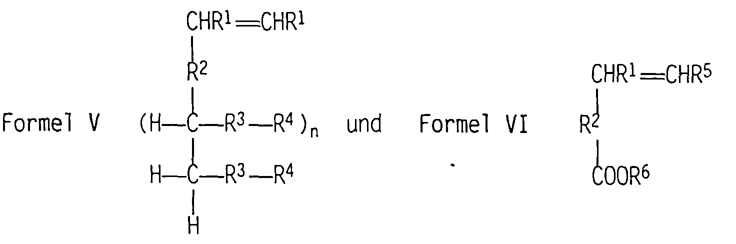 sind, wobei
R¹ Wasserstoff oder den Methylrest,
R² einen zweiwertigen organischen Rest, vorzugsweise einen aliphatischen, cycloaliphatischen oder aromatischen Kohlenwasserstoffrest mit bis zu 10 Kohlenstoffatomen, oder eine C-C-Einfachbindung,
R³ -O- oder -NH-,
R⁴ Wasserstoff oder den Rest -SO₃⁻Na⁺,
R⁵ Wasserstoff, den Methylrest oder den Rest -R²-COOR⁶,
R⁶ Wasserstoff oder Na bedeuten und
n für 4 oder 5 steht;
mit der Maßgabe, daß mindestens einer der Substituenten R⁴ ein Rest -SO₃⁻Na⁺ ist.

9. Verfahren nach einem der Ansprüche 1 bis 8, dadurch gekennzeichnet, daß der Komplexbildner ein Chelatkomplexbildner ist.

10. Verfahren nach Anspruch 9, dadurch gekennzeichnet, daß der Chelatkomplexbildner Nitrilotriessigsäure, Ethylendiamintetraessigsäure oder ein saures oder neutrales Alkali- oder Eralkalimetallsalz dieser Säuren ist.

11. Erzeugnisse für technische, biotechnische oder insbesondere medizinische Zwecke oder Teile solcher Erzeugnisse, deren Oberflächen nach dem Verfahren eines der Ansprüche 1 bis 10 behandelt wurden.

12. Verwendung eines Erzeugnisses, das nach dem Verfahren eines der Ansprüche 1 bis 10 behandelt wurde, für die Herstellung von Apparaten, Geräten und Vorrichtungen für technische, biotechnische oder insbesondere medizinische Zwecke.
